# EUROPÄISCHE PATENTANMELDUNG

(11) **EP 2 085 469 A1**
(43) Veröffentlichungstag der Anmeldung: **05.08.2009**
(21) Anmeldenummer: 09001176.8
(22) Anmeldetag: 28.01.2009
(51) Int. Cl.: C12N 9/20, C12P 7/64, C01F 7/02

(54) **Lipasenformulierung**

(30) Priorität: 30.01.2008 DE 102008006716
(71) Anmelder: BAM Bundesanstalt für Materialforschung und- Prüfung, 12205 Berlin (DE)
(72) Erfinder: Brenneis, Rudolf, Dr. rer.nat., 10315 Berlin (DE); Baeck, Burkhard, Dipl.-Ing. (FH), 10551 Berlin (DE); Löffler, Christel, Dipl.-Chem., 12679 Berlin (DE); Kley, Gerd, Dr. rer.nat., 16727 Oberkrämer (DE)
(74) Vertreter: Pfenning, Meinig & Partner GbR

(57) **Zusammenfassung**

Vorliegende Erfindung betrifft eine Lipasenformulierung, die neben einer in einem organischen Lösungsmittel gelösten Lipase gemahlene Übergangstonerden mit einem mittleren Korndurchmesser < 0,5 mm enthält. Eine derartige Lipasenformulierung erlaubt die Katalyse einer Vielzahl von Reaktionen, beispielsweise die Alkoholyse von Fetten, die Veresterung von Fettsäuren oder eine parallel ablaufende Alkoholyse von Gemischen aus Tri-, Di- und Monoglyceriden neben der Veresterung von Fettsäuren.

## Beschreibung

Vorliegende Erfindung betrifft eine Lipasenformulierung, die neben einer in einem organischen Lösungsmittel gelösten Lipase gemahlenes Aluminiumhydroxid in Form von Böhmit (γ-AlO(OH)), mit einem mittleren Korndurchmesser < 0,5 mm enthält. Eine derartige Lipasenformulierung erlaubt die Katalyse einer Vielzahl von Reaktionen, beispielsweise die Alkoholyse von Fetten, die Veresterung von Fettsäuren oder eine parallel ablaufende Alkoholyse von Gemischen aus Tri-, Di- und Monoglyceriden neben der Veresterung von Fettsäuren.

Die wichtigsten nachwachsenden Rohstoffe der chemischen Industrie sind Fette und Öle (H. Baumann, M. Bühler, H. Fochem, F. Hirsinger, H. Zoebelein, J. Falbe (1988): "Natürliche Fette und Öle - Nachwachsende Rohstoffe für die chemische Industrie". Angew. Chem. 100, 4 1-62; J.O. Metzger, U. Bornscheuer (2006): "Lipids as renewable resources: current state of chemical and biotechnological conversion and diversification", Appl. Microbiol. Biotechnol. 71, 13-22). Aufbereitete Fettrohstoffe können durch chemische Grundprozesse unmittelbar in Zwischen- und Endprodukte überführt werden, wobei deren Einsatzmöglichkeiten von Nahrungsmitteln über Kosmetik- und Waschmittelrohstoffe und Kunststoffe bis zu Treibstoffen (Biodiesel) oder ökologisch verträglichen Schmierstoffen reichen (C. Breucker, V. Jordan, M. Nitsche, B. Gutsche (1995): "Oleochemie - Chemieprodukte auf der Basis nachwachsender Rohstoffe. Chem.-Ing.-Techn. 67, 430-440).

Bedingt durch ein nachhaltiges Wirtschaften mit den vorhandenen Ressourcen und eine Erschließung umweltfreundlicher Rohstoffe und Technologien gewinnt die Nutzung nachwachsender Rohstoffe zunehmend an Bedeutung (z.B. die Herstellung von Biodiesel auf der Basis von Rapsöl als Ersatz für mineralischen Dieselkraftstoff). Darüber hinaus wird in der metallverarbeitenden Industrie der Versuch unternommen, die aus ökologischen Gründen (schlechte Umweltverträglichkeit) und aus negativen arbeitsphysiologischen Gründen (Gesundheitsgefährdung durch Kontakt mit entstehenden Ölnebeln bzw. Öldämpfen) bedenkliche mineralölbasierten Kühlschmierstoffe durch Produkte auf der Basis von natürlich vorkommenden Ölen und Fetten zu ersetzen. So wurden z.B. Monoalkylester auf der Basis von hoch-raffinierter Palmitinsäure entwickelt und als Kühlschmierstoff getestet.

Trotz ökologischer und auch technologischer Vorteile, z.B. besserer Schmierwirkung gegenüber mineralölbasierten Kühlschmierstoffen, werden Kühlschmierstoffe auf Basis von Fettsäureestern in der Praxis kaum eingesetzt, da ihre Anschaffungskosten, die aus dem Syntheseaufwand und den Rohstoffpreisen resultieren, weit über denen von konventionellen Produkten auf Mineralölbasis liegen. Um den Einsatz aus genannten Gründen zu forcieren, müssen preisgünstige Alternativen sowohl hinsichtlich des lipiden Ausgangsmaterials als auch des Syntheseweges aufgezeigt werden.

Es gibt folgende zwei Möglichkeiten um eine Preisreduzierung für das Endprodukt Kühlschmierstoff auf Basis von Fettsäureestern zu erzielen:
a) durch den Einsatz von bei der Tierkörperverwertung oder der Altspeisefettsammlung gewonnenen Fetten als Rohstoffe,
b) durch die Entwicklung von preisgünstigen, alternativen chemischen Synthesewegen.

Natürliche Fette und Öle sind nachwachsende und - als Quelle von Fettsäuren - gefragte Rohstoffe für die chemische Industrie. Die durch chemische oder physikalische Raffination gereinigten nativen Fette und Öle werden in oleochemischen Grundprozessen durch Hydrolyse, Veresterung und/oder Umesterung umgesetzt. Bei der Hydrolyse mit Wasserdampf werden Fette und Öle in Glycerin und Fettsäuren gespalten. Die Veresterung von Fettsäuren mit Alkoholen führt zu Fettsäureestern. Durch eine gezielte Wahl des Alkohols lassen sich spezielle Fettsäureester herstellen. Die Umesterung ist die Umsetzung von Fetten und Ölen mit Alkoholen (Alkoholyse), Fettsäuren (Acidolyse), Fettsäureestern oder anderen Fetten und Ölen. Bei der Alkoholyse wird der Alkohol bzw. das Alkanol im Überschuss zugesetzt, um eine hohe Ausbeute am gewünschten Ester zu erhalten. Die Umesterung ermöglicht den Zugang zu zahlreichen oleochemischen Produkten des Non-Food- und Food-Bereichs. Zurzeit ist die Umesterung die am wenigsten eingesetzte Technologie zur Fettmodifikation.

Es gibt Bestrebungen, gereinigte (Filtration, Entwässerung) Altspeise- und Tierfette einer direkten stofflichen Verwertung zuzuführen, zumal der Gehalt an Triglyceriden, z.B. in Altspeisefetten über 96 % liegt und spurenanalytische Untersuchungen die niedrige Grundbelastung mit anorganischen und organischen Schadstoffen belegen (M. Bahadir, R. Bock, T. Dettmer, O. Falk, J. Hesselbach, P. Jopke, R. Meyer-Pitroff, C. Schmidt-Nädler, H. Wichmann (2003): "Chemisch-analytische Charakterisierung technischen tirischen Fettes aus einer Tierkörperbeseitigungsanstalt", UWSF - Z. Umweltchem. Ökotox. 15, 19-28; O. Falk, H. Wichmann, P. Jopke, C. Schmidt-Nädler, B. Matthies, M. Bahadir, R. Meyer-Pitroff (2004): "Verbleib von Spurenschadstoffen bei der Methylesterherstellung aus Altspeisefett im Technikumsmaßstab", UWSF - Z. Umweltchem. Ökotox. 16, 255-261). Weiterhin ist bekannt, dass Enzyme als Biokatalysatoren chemische Reaktionen beschleunigen bzw. selektieren können.

Dabei wird die Fähigkeit von Enzymen an zahlreichen Substanzen in spezifischer Weise Reaktionen, wie z.B. Oxidationen, Hydrierungen, Hydrolysen, Methylierungen oder Veresterungen durchzuführen, ausgenutzt. Dabei können auch mehrere enzymatisch katalysierte Reaktionsschritte miteinander kombiniert sein. Das Einsatzgebiet von Enzymen reicht von der Textilindustrie, Lederherstellung, Pelzzurichtung und -veredelung, Zellstoff- und Papierherstellung über die Futtermittelindustrie und umweltschutzrelevante Bereiche (Abwasseraufbereitung) bis zur Verwendung in der modernen Analytik (Lebensmittelproduktion und -überwachung, medizinische Diagnostik) und für therapeutische Zwecke. Unter den für industrielle Zwecke eingesetzten Enzymklassen sind die Hydrolasen von besonderer Bedeutung. Hydrolasen umfassen eine Gruppe von Enzymen, die chemische Bindungen durch die Reaktion mit Wasser spalten. Hydrolasen bilden die 3. Hauptgruppe (EC 3) der Enzymklassifizierung der International Union of Biochemistry und werden nach der Art der hydrolysierten chemischen Bindung in weitere Gruppen unterteilt. Esterasen (EC 3.1.1.x) stellen eine diverse Gruppe von Hydrolasen dar, die sowohl Bildung als auch Hydrolyse von Esterbindungen katalysieren und bei Pflanzen, Tieren und Mikroorganismen weit verbreitet sind. Echte Lipasen (Triacylglycerolester-Hydrolasen, EC 3.1.1.3) katalysieren sowohl die Hydrolyse als auch die Synthese von Estern aus Glycerin und langkettigen Fettsäuren. Ihre bevorzugten Substrate sind wasserunlösliche langkettige Triacylglyceride, die an der Interphase zwischen hydrophober Lipid- und hydrophiler Wasserphase zu Di- und Monoglyceriden, Fettsäuren und Glycerin umgesetzt werden. Lipasen werden aus Mikroorganismen, Pilzen und Pflanzen gewonnen (z.B. die Lipasen aus *Candida antarctica*, Novozym 735 L der Fa. Novozymes A/S in Dänemark, oder *Humicola lanuginosa* etc.). Neben der Hydrolyse und Synthese von Triglyceriden können von Lipasen auch weitere Reaktionen katalysiert werden, wie z.B. die Veresterung primärer, sekundärer und tertiärer Alkohole, die Umesterung von Lipiden, Transesterifikationen (Glycerolyse bzw. Alkoholyse) und Interesterifikationen. Die Lipasen werden von unterschiedlichen Firmen angeboten, wobei Firmen, wie Fluka, Amano, Novozymes und Meito den Enzymmarkt weitgehend abdecken. Etwa 40 % des Weltmarktes werden von der dänischen Firma Novozymes A/S in Bagsværd beliefert, Amano ist neben Meito Marktführer im asiatischen Raum und Fluka hat große Bedeutung in Mitteleuropa.

Enzyme tragen in chemischen Produktionsverfahren zu einer besseren Ausnutzung der Rohstoffe, zu einer Herabsetzung des Energieverbrauchs und zu einer verbesserten Produktqualität bei. Das hat zur Folge, dass in den letzten Jahren die Anwendung der enzymatischen Katalyse und der Bioreaktortechnik auch auf die oleochemischen Grundreaktionen Hydrolyse, Veresterung und Umesterung intensiv untersucht wurde (U. Bornscheuer (Hrsg) (2000): "Enzymes in Lipid Modification" Wiley-VCH Verlag GmbH Weinheim). Diese drei Reaktionstypen sind durch die folgenden Reaktionsschemata wiedergegeben.

### Hydrolyse:

Fett + Wasser → Fettsäuren + Glycerol (1)

### Veresterung:

Fettsäuren + Alkohol → Fettsäureester + Wasser (2)

### Umesterung (Alkoholyse):

Fett + Alkohol → Fettsäureester + Glycerol (3)

Grundsätzlich können Fettsäureester (Esteröle), wie aus den Gleichungen (1) bis (3) hervorgeht, auf zwei Wegen hergestellt werden:
- über ein zweistufiges Verfahren (Hydrolyse + Veresterung) entsprechend den Gleichungen (1) + (2) oder
- über ein einstufiges Verfahren (Alkoholyse) entsprechend Gleichung (3).

Entgegen der weitläufigen Meinung, dass eine enzymatische Alkoholyse nur unter Ausschluss bzw. in Gegenwart lediglich von Spuren von Wasser möglich ist, konnte durch Untersuchungen gezeigt werden, dass beim Einsatz der nativen Lipase A von *Candida antarctica* die Alkoholyse in Anwesenheit größerer Wassermengen und nichtwassermischbarer Alkohole die bevorzugte Reaktion gegenüber der Hydrolyse ist. Bei dem in Rührreaktoren durchgeführten Prozess dient das Wasser als "Trägerphase" für das Enzym und das entstehende Glycerol, bewirkt aber auch, dass durch eine parallel ablaufende enzymatische Hydrolyse bestimmte Mengen an Fettsäuren (< 5 %) entstehen (R. Brenneis, B. Baeck, G. Kley (2004): "Alcoholysis of waste fats with 2-ethyl-1-hexanol using Candida Antarctica lipase A in large-scale tests". Eur. J. Lipid Sci. Technol. 106, 809-814). Die praktischen Auswirkungen eines zu hohen Gehaltes an freien Fettsäuren im Fettsäureester-Produkt seien beispielhaft kurz erläutert. In einem Schleifversuch (PKW-Kurbelwellenproduktion) wurde ein aus Altfett enzymkatalysiert hergestellter 2-Ethylhexylester mit einem Gehalt an freien Fettsäuren von rund 5 % eingesetzt. Vom Schleifprozess her bereitete der Versuch keine Probleme. Der Schleifprozess musste jedoch nach der Verstopfung eines Filters abgebrochen werden. Das dort verwendete Filtermaterial aus Cellulose stellte eine Calciumquelle dar, so dass die freien Fettsäuren als Ca-Salze (Seife) ausfielen (R. Brenneis, H. Wichmann, R. Sprenger, A. Eickhoff, R. Bock, T. Dettmer, B. Baeck, C. Reger, M. Bahadir (2006): "Nativ-basierte Grundöle für die Produktion von Kühlschmierstoffen - Stand der Entwicklung und Defizite". UWSF - Z. Umweltchem. Ökotox. 18, 95-101).

Bei der enzymatischen Alkoholyse werden Fett- und Alkoholkomponenten im molaren Verhältnis eingesetzt. Durch die parallel ablaufende Hydrolyse entstehen dabei neben dem Hauptprodukt "Fettsäure-2-Ethylhexyl-ester" bestimmte Mengen an Fettsäuren. Somit verbleibt der diesen Fettsäuren adäquate Anteil an 2-Ethyl-1-Hexanol im Produkt und muss in einem nachgeschalteten Schritt abdestilliert werden. Theoretisch sollte es möglich sein, in einer zweiten enzymatisch katalysierten Reaktion die freien Fettsäuren mit dem Restalkohol entsprechend Gleichung (2) zu verestern.

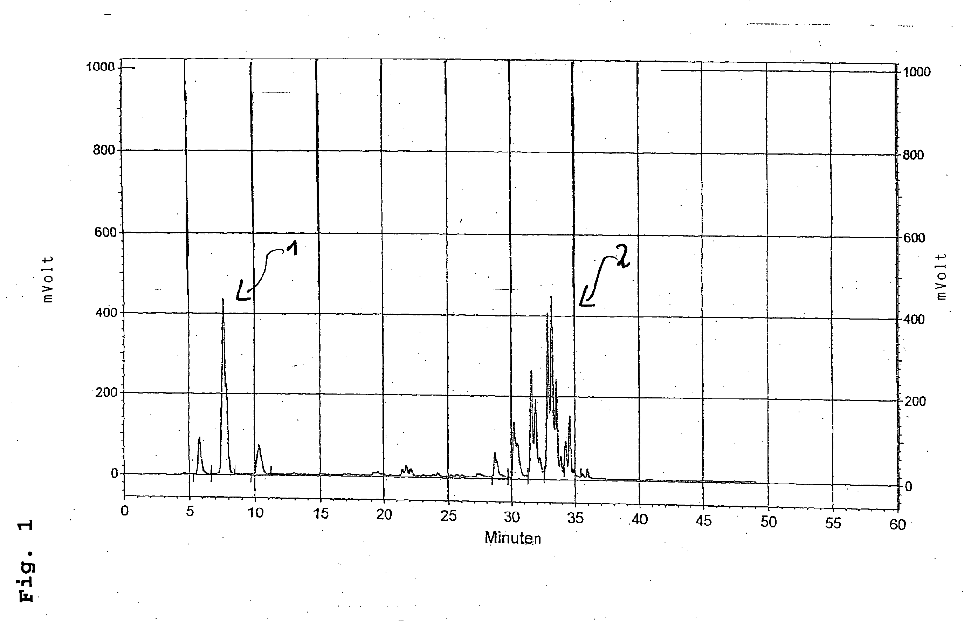

Bei dem zu entwickelnden enzymkatalysiert arbeitenden Verfahren zur Esterherstellung sollen aus Kostengründen lipide Ausgangsstoffe niederer Qualität eingesetzt werden. Kennzeichnend für diese Stoffe ist, dass sie neben den Fetten (Triglyceriden) einen mehr oder weniger hohen Anteil an freien Fettsäuren enthalten können. Fig. 1 zeigt das HPLC-Spektrum eines solchen Ausgangsmaterials. Es ist ersichtlich, dass neben Fett in fast gleichen Mengenanteilen freie Fettsäuren vorliegen.

Um diese Komponenten mit höhermolekularen Alkoholen (nichtwassermischbar) umzusetzen, sind folgende zweistufige Verfahren A) oder B) denkbar, die beide eine "Veresterungsstufe" beinhalten:
**A)**
   **1. Stufe (Hydrolyse):**

      Fett + Fettsäuren I + Wasser → Fettsäuren I + Fettsäuren H + Glycerol
   **2. Stufe (Veresterung):**

      Fettsäuren (I +H) + Alkohol

      → Fettsäureester V + Wasser.
**B)**
   **1. Stufe (Alkoholyse):**

      Fett + Fettsäuren I + Alkohol → Fettsäureester A + Fettsäuren I + Glycerol
   **2. Stufe (Veresterung):**

      Fettsäuren I + Alkohol + Fettsäureester A → Fettsäureester (A+V) + Wasser

### Legende zu A) und B)

- Freie Fettsäuren aus dem Fett/Fettsäuregemisch = Fettsäuren I,
- durch Hydrolyse des Fettes entstandene Fettsäuren = Fettsäuren H,
- durch Alkoholyse entstandene Fettsäureester = Fettsäureester A,
- durch Veresterung entstandene Fettsäureester = Fettsäureester V

Sowohl Variante A) als auch Variante B) wurden untersucht. Problematisch bei enzymatisch katalysierten Veresterungsreaktionen ist Folgendes: Für eine Aktivierung der Lipasen (Enzyme für fettmodifizierende Reaktionen) muss sich ein "lid" (Deckel) öffnen, unter dem sich das aktive Zentrum befindet. Das ist aber nur bei Anwesenheit von Wasser möglich. Andererseits ist Wasser bei einer Veresterungsreaktion unerwünscht, weil es das chemische Gleichgewicht

Säure + Alkohol ↔ Ester + Wasser

in Richtung Ausgangsstoffe verschiebt. Um hohe Ausbeuten zu erhalten, muss das entstehende Wasser aus dem Gleichgewicht entfernt werden. Hierfür werden in der Literatur unterschiedliche Methoden angeführt (X. Xu (2003): "Engineering of enzymatic reactions and reactors for lipid modification and synthesis". Eur. J. Lipid Sci. Technol. 105, 289-304):
- Verdampfung im Vakuum,
- Externe Trockenmittel (Destillation azeotroper Gemische),
- Interne Trockenmittel (Molekularsiebe, Silicagel),
- Durchleiten von Luft oder Stickstoff,
- Membransysteme,
- Einsatz hydrophiler Lösungsmittel,
- Einsatz von Salzen unterschiedlicher Wasseraktivität.

In der Patentliteratur wird an zahlreichen Stellen über die Verwendung von *Candida antarctica* A berichtet (siehe z.B. DE 10 2004 044 660 A1, DE 10 2005 002 700 A1, WO 90/13 656 A1, WO 90/04 033 A1, EP 1 358 306 B1. In WO 90/13 656 A1 und in WO 90/04 033 A1 wird die Herstellung von Monoglyceriden über enzymatische Alkoholyse mit verschiedenen Alkoholen und wenig Wasser im Ansatz beschrieben. Dabei werden Lipasen in Pulverform oder immobilisiert eingesetzt.

Hiervon ausgehend ist es die Aufgabe der vorliegenden Erfindung, eine katalytisch wirkende Zusammensetzung bereitzustellen, die einen vielseitigen Einsatz sowohl bei der Veresterung oder Umesterung als auch bei der Alkoholyse von Fetten erlaubt und die ein einstufiges, enzymatisch-katalytisches Verfahren zur preisgünstigen Herstellung von Fettsäureestern aus Fetten, Fettsäuren oder Fett-Fettsäure-Gemischen ermöglicht.

Diese Aufgabe wird mit der Lipasenformulierung mit den Merkmalen des Patentanspruchs 1 gelöst. Die abhängigen Ansprüche stellen dabei vorteilhafte Weiterbildungen dar. Mit den Patentansprüchen 8 bis 11 werden Verwendungszwecke der Lipasenformulierung angegeben.

Erfindungsgemäß wird eine Lipasenformulierung bereitgestellt, die mindestens eine Lipase in einem organischen Lösungsmittel und gemahlenes Aluminiumhydroxid, dessen Kristallstruktur im Wesentlichen der des Böhmits (γ-AlO(OH) entspricht, mit einem mittleren Korndurchmesser kleiner 0,5 mm enthält.

Mit dieser erfindungsgemäßen Lipasenformulierung wird überraschenderweise ein im Wesentlichen einstufiges, enzymatisch-katalytisches Verfahren zur preisgünstigen Herstellung von Fettsäureestern aus Fetten, Fettsäuren oder Fett/Fettsäure-Gemischen ermöglicht. Dabei werden die katalytischen Wirkungen von Lipasen, wie z.B. *Candida antarctica* A und B, *Thermomyces lanuginosus* und/oder *Aspergillus orycae* auf Alkoholyse von Fetten sowie auf Veresterung von Fettsäuren so optimiert, dass im Rahmen eines einstufigen Verfahrens gleichzeitig die Alkoholyse von Fetten sowie die Veresterung von Fettsäuren erfolgen kann.

Vorzugsweise beträgt die Lipasenaktivität der Lipasenformulierung zwischen 5 und 100 KLU/g.

Bezüglich der Konzentration des Böhmits ist es bevorzugt, wenn diese, bezogen auf 1 bis 5 ml Lipaselösung 1 bis 10 g an Böhmit in gemahlener Form beträgt. Der Anteil der verwendeten Lipaselösung wird dabei so gewählt, dass diese durch die eingesetzte Menge an Böhmit vollständig aufgesaugt wird, so dass kein zusätzlicher Schritt zur Abtrennung von überschüssiger Lipaselösung erforderlich ist.

Als bevorzugte Lipase wird in der Lipasenformulierung *Candida antarctica* (A-Lipase) eingesetzt. Eine besonders geeignete gebrauchsfertige Lipaselösung ist z.B. bei Novo Nordisk, Dänemark unter dem Markennamen Novozym 868 L^{®} bzw. Novozym 735^{®} bzw. Novocor ADL^{®} erhältlich.

Weiter bevorzugt ist es, wenn die Lipasenformulierung zusätzlich *Thermomyces lanuginosus* und/oder *Candida antarctica* (B-Lipase) und/oder *Aspergillus oryzae* enthält.

Die bevorzugten mittleren Korndurchmesser des eingesetzten gemahlenen Böhmits beträgt dabei weniger als 0,2 mm, weiter bevorzugt weniger als 0,1 mm, insbesondere weniger als 0,07 mm.

Eine für die erfindungsgemäße Lipasenformulierung eingesetztes aktivierte Aluminiumhydroxid kann z.B. dadurch hergestellt werden, dass auf Basis von Aluminiumhydroxid als Rohmaterialien unter kontrollierten Bedingungen eine thermische Behandlung bzw. Aktivierung erfolgt, um einen Großteil des gebundenen Kristallwassers zu eliminieren.

Hierdurch findet ein Umbau des Kristallgitters statt. Die neu entstehende Kristallphase ist dabei überwiegend Böhmit. Das eingesetzte aktivierte Böhmit zeichnet sich durch folgende spezifische Merkmale aus:
- hohe spezifische Oberfläche,
- großes Porenvolumen,
- definierte Porengrößenverteilung,
- spezifische katalytische Aktivität,
- spezifische adsorptive Kapazität und
- hohe Bruchfestigkeit und damit einen sehr geringen Staubgehalt.

Bevorzugt weist das eingesetzte Aluminiumhydroxid [AlO(OH)] eine spezifische Oberfläche (BET) in (m²/g) von 200 bis 260, ein Porenvolumen in (cm³/g) von ca. 0,35 und eine Schüttdichte in (kg/m³) von 850 bis 950 auf.

Ein vorstehend beschriebenes aktiviertes Böhmit kann z.B. bei den Martinswerken GmbH in Kölner Straße 110, 50127 Bergheim, Deutschland unter dem Markennamen COMPALOX^{®} bezogen werden.

Die Erfindung betrifft somit die Bereitstellung eines katalytischen Systems, enthaltend *Candida antarctica* A sowie Böhmit, zur Entwicklung eines vorteilhaften einstufigen enzymatisch-katalytischen Verfahrens gegenüber den mehrstufigen Verfahren (Alkoholyse der Fette mit anschließender Veresterung der Fettsäuren).

Wie bereits weiter oben ausgeführt, ist es aus ökologischen Gründen und aus arbeitsphysiologischen Gründen wünschenswert, mineralölbasierte Kühlschmierstoffe durch Produkte auf Basis von natürlich vorkommenden Ölen und Fetten zu ersetzen. Esterbasierte Schmierstoffe auf Basis nachwachsender Rohstoffe werden üblicherweise durch Veresterung von Fettsäuren mit den entsprechenden Alkoholen hergestellt. Zu diesem Zweck muss erst Fett unter hohen Temperaturen und hohem Druck in Fettsäuren und Glycerin gespalten werden. Anschließend werden die Fettsäuren unter hohem Energieeinsatz verestert. Der hohe Preis für Schmierstoffe auf Basis nachwachsender Rohstoffe ist einerseits in dem hohen Syntheseaufwand begründet, andererseits in den Rohstoffpreisen. Daher wurde bei der hier vorliegenden Erfindung der Versuch unternommen, eine katalytische Formulierung zu entwickeln, mit der einerseits preisgünstige lipide Ausgangstoffe (z.B.

Altspeise- und Tierfette) verwendet und anderseits die Reaktionszeiten verkürzt werden können. In dieser Hinsicht bietet die erfindungsgemäße Lipasenformulierung eine neue vorteilhafte Möglichkeit, um Altfettsäureester zum Einsatz als Kühlschmierstoffe preiswert herzustellen.

Die vorliegende Erfindung wird anhand der nachfolgenden beispielhaften Ausführungen näher erläutert, ohne die Erfindung auf die genannten speziellen Parameter zu beschränken.

### Generelles Verfahren zur Herstellung der Lipasenformulierung

Eine enzymatisch katalysierte Veresterung von Fettsäuren durch nichtwassermischbare Alkohole entsprechend Gleichung (2) lässt sich folgendermaßen durchführen:

Das Flüssigpräparat der Lipase (wie es von den Enzymherstellern geliefert wird), wird vor ihrem Einsatz mit Aluminiumhydroxid (Böhmit) gemischt. Das eingesetzte Böhmit muss eine Korngröße < 0,2 mm aufweisen. Hierzu ist es erforderlich, dass das käuflich erworbene Böhmit, wie Compalox^{®} von den Martinswerken, da es in größerem Durchmesser geliefert wird, in einen Mörser, bevorzugt in einem Korundmörser, auf den angegebenen Korndurchmesser von < 0,2 mm gemahlen wird.

Nach der Herstellung der Mischung bleibt diese etwa 0,5 h stehen und wird dann den Fettsäure/Alkohol-Gemischen zugesetzt. Die getesteten Lipasen von
- *Thermomyces lanuginosus*
- *Candida antarctica*, A
- *Candida antarctica,* B
- *Aspergillus oryzae*
waren in der Enzym/Böhmit-Mischung in der Lage, eine Veresterung im Rührreaktor bis zum vollständigen Umsatz zu katalysieren. Spuren von im Böhmit adsorbiertem Wasser sind somit in der Lage, die Lipasen zu aktivieren; das Böhmit kann außerdem das Reaktionsprodukt Wasser aufnehmen.

Darüber hinaus wurde überraschenderweise gefunden, dass die Enzym/Böhmit-Mischung der A-Lipase von *Candida antarctica* in der Lage ist, auch eine Alkoholyse entsprechend Gleichung (3) durchzuführen. Das Böhmit kann also auch das bei dieser Reaktion entstehende Glycerol aufnehmen bzw. binden.

Ebenso wurde überraschenderweise gefunden, dass die Lipase A von *Candida antarctica* in der Lage ist, zwei unterschiedliche Reaktionen parallel zu katalysieren, so dass es nun möglich ist, mit dieser Enzym/Böhmit-Mischung den unter B) dargestellten zweistufigen Prozess (Alkoholyse der Fette mit anschließender Veresterung der Fettsäuren) einstufig durchzuführen.

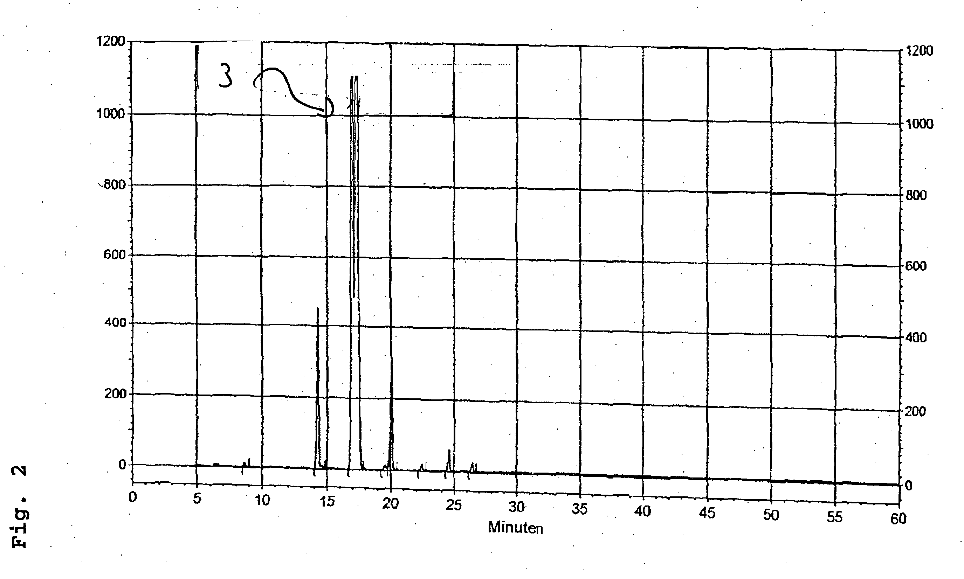

Wie aus Fig. 1, die das HPLC-Spektrum eines preiswerten lipiden Ausgangsmaterials zeigt und Fig. 2, die das Produkt der Umsetzung dieses Fett/Fettsäure-Gemisches mit 2-Ethyl-1-Hexanol unter Einsatz der Lipase A von *Candida antarctica* in Mischung mit dem Böhmit zeigt, hervorgeht, ist dieses möglich. Detaillierte Untersuchungen ergaben, dass die Alkoholyse aber wesentlich schneller abläuft als die Veresterung.

In Figur 1, dem HPLC-Spektrum des Ausgangsmaterials, ist mit 1 die Fettsäure und 2 das Fett bezeichnet. In Figur 2 ist der Fettsäureester durch 3 dargestellt.

Vergleichende Untersuchungen mit den drei o.a. Lipasen zu Veresterungsreaktionen hatten gezeigt, dass *Thermomyces lanuginosus*/Böhmit-Mischungen die höchsten Umsatzraten zeigten und diese Lipase außerdem die preiswerteste ist. Es stellte sich somit die Frage, ob bei Einsatz eines Fett/Fettsäure-Gemisches als Ausgangsmaterial nicht ein Teil der Lipase A von Can*dida antarctica* durch *Thermomyces lanuginosus* ersetzt werden kann, um somit eine gleichzeitig ablaufende Veresterung und Alkoholyse zu ermöglichen. Da nur *Candida antarctica* (A-Lipase) eine Alkoholyse katalysieren kann, würde sie diesen Part übernehmen. Die Veresterung würde von der für diese Reaktion prädestinierten *Thermomyces lanuginosus* sowie partiell von *Candida antarctica* übernommen.

Auch diese Versuche erwiesen sich als erfolgreich, d.h. die Reaktionszeiten bis zum vollständigen Umsatz lassen sich noch verkürzen und der Prozess ist preiswerter durchführbar.

Kurz zusammengefasst handelt es sich praktisch um folgende neuen Erkenntnisse:
- Gemahlenes Aluminiumhydroxid in Form von Böhmit [(γ-AlO(OH))] (genauer: Spuren von Wasser, die in ihnen gebunden sind) sind in der Lage, native Lipasen zu aktivieren.
- Die Mischung *Candida antarctica* (A-Lipase)/Böhmit kann eine Alkoholyse von Fetten katalysieren, ohne dass es dabei zur Nebenreaktion Hydrolyse unter Bildung von freien Fettsäuren kommt.
- Die Mischungen *Thermomyces lanuginosus, Candida antarctica* (A), *Candida antarctica* (B) und/oder *Aspergillus orycae* mit Böhmit kann Veresterungen von Fettsäuren katalysieren.
- Die Mischung *Candida antarctica* (A-Lipase)/Böhmit kann parallel eine Alkoholyse von Fetten und eine Veresterung von Fettsäuren katalysieren. Die Umsatzrate der parallel ablaufenden Veresterung kann gesteigert werden, wenn *Candida antarctica* partiell durch *Thermomyces lanuginosus* ersetzt wird.

### Ausführungsbeispiel

### Verfahren zur Herstellung von Fettsäureestern aus Fetten, Fett/Fettsäure-Gemischen oder Fettsäuren

### (Durchführung der Reaktionen bei 70 °C in Rührreaktoren unter Einsatz von Candida antarctica, Lipase A)

### Voraussetzung:

die Alkoholkomponente ist nicht mit Wasser mischbar

### A) Einsatz von (Alt-)Fetten mit niederen Fettsäuregehalten (z.B. Umsetzung mit 2-Ethyl-1-Hexanol)

### 1. Alkoholyse

Fett und Alkohol werden in den Reaktor gefüllt (der Alkohol sollte im leichten Überschuss vorliegen) und gut durchgerührt. Dazu wird Wasser (mengenmäßig etwa wie die Fettmenge) gegeben. Die Mischung wird auf 70 °C erwärmt. Zum Enzym wird etwas Wasser zugesetzt. Diese Lösung wird dann dem Reaktor zugesetzt, wonach die enzymkatalysierten Reaktionen beginnen.

### Hauptreaktion:

Fett + 2-Ethyl-1-Hexanol → Fettsäure-2-ethylhexylester + Glycerol

### Nebenreaktion:

Fett + Wasser → Fettsäuren + Glycerol

Nach Beendigung der Umsetzungen (der Umsetzungsverlauf wird durch HPLC-Messungen von entnommenen Proben verfolgt) wird das Rührwerk ausgeschaltet. Im Reaktor trennen sich zwei Phasen:
- Oben:: Fettsäure-2-ethylhexylester/Fettsäuren/2-Ethyl-1-Hexanol
- Unten:: Wasser/Glycerol/Enzym

Die Fettsäuren in der oberen Phase sind aus dem Ausgangsfett bzw. durch die Nebenreaktion entstanden. 2-Ethyl-1-Hexanol ist enthalten, weil es zum einen im Überschuss zugegeben wurde, zum zweiten durch die Nebenreaktion einiges nicht in den Ester umgesetzt worden ist. Da es schwierig ist, zu Beginn ein molares Fett/Alkohol-Verhältnis einzustellen, wird Alkohol im Überschuss zugegeben. Sowohl Fettsäuren als auch überschüssiger Alkohol stören im Esterprodukt (der Alkohol ließe sich kostspielig durch Vakuumdestillation entfernen). Deshalb schließen sich die folgenden enzymkatalysierten Reaktionen an.

### 2. Veresterung

Böhmit (gebraucht, aus der Wasserstoffperoxidproduktion oder neu) wird gemahlen (praktikable mittlere Korngrößenverteilung < 0,2 mm) und *Candida* antarctica, Lipase A (nativ, in Lösung) werden gut gemischt und ca. 0,5 h stehengelassen. Hierbei wird das Enzym durch das in dem Böhmit enthaltene Wasser aktiviert.

Die obere Phase (Ester/Fettsäure/Alkohol-Gemisch) aus dem ersten Reaktor (Alkoholyse) wird in den zweiten Reaktor gefüllt und auf 70 °C unter Rühren erwärmt. Dann wird das Böhmit/Enzym-Gemisch zugesetzt. Die im Ester enthaltenen Fettsäuren werden mit dem enthaltenen 2-Ethyl-1-Hexanol verestert:

Fettsäuren + 2-Ethyl-1-Hexanol → Fettsäure-2-ethylhexylester + Wasser

Das entstehende Wasser wird von dem Böhmit aufgenommen. Wenn alle Fettsäuren umgesetzt sind, muss der überschüssige Alkohol, der einen leicht stechenden Geruch hat, umgesetzt werden. Das geschieht im gleichen Reaktor, ohne dass dieser abgestellt wird.

### 3. Umesterung

Dem Reaktor wird eine kleine Menge Fettsäuremethylester (Biodiesel) zugesetzt. Es findet folgende enzymkatalysierte Reaktion statt:

Fettsäuremethylester + 2-Ethyl-1-Hexanol → Fettsäure-2-ethylhexylester + Methanol

Das Methanol hat einen Siedepunkt von 68 °C und verdampft. Das Endprodukt besteht jetzt nur aus Fettsäure-2-ethylhexylester und geringen, nicht umgesetzten Anteilen des Biodiesels, die die (Anwendungs-)Eigenschaften des Produktes auf Grund seiner gleichen chemischen Struktur (Fettsäureester) nicht oder nicht relevant beeinflussen.

Nach Beendigung der Reaktion und Ausschalten des Rührwerks separieren 2 Phasen.
Oben: Fettsäure-2-Ethylhexylester/Fettsäuremethylester
Unten: Böhmit/Enzym/Wasser

Nach Abziehen der oberen Phase (Produkt) wird der zweite Reaktor zum ersten Reaktor, d.h. in ihm findet die nächste Alkoholysereaktion (s.o.) statt. Hierzu wird soviel Wasser in den Reaktor gegeben, wie für die Alkoholysereaktion benötigt wird. Dazu kommen dann Fett und Alkohol. Enzym muss nicht neu zugesetzt werden. Es hat sich gezeigt, dass die Anwesenheit des Böhmits bei der Alkoholyse (wo sie ja eigentlich keine Funktion hat) die Reaktionszeiten verkürzt. Offenbar bewirken diese feinen Partikel eine Verbesserung der Durchmischung der Reaktionskomponenten.

Neues Enzym muss bei der folgenden Produktionskette somit nur jeweils vor der Veresterungsreaktion eingesetzt werden.

### B. Einsatz von Fett/Fettsäuregemischen bzw. Fettsäuren

Dieses einstufige Verfahren kann auch auf reine Fette angewandt werden.

Wie bei der Veresterungsreaktion werden Böhmit und Enzymlösung gemischt (größere Mengen) und dem Reaktor, in dem das lipide Gemisch und 2-Ethyl-1-Hexanol (letzteres im leichten Überschuss) unter Rühren auf 70 °C erwärmt werden, zugesetzt.

Es finden parallel Alkoholyse und Veresterung statt:

Fett + 2-Ethyl-1-Hexanol → Fettsäure-2-ethylhexylester + Glycerol

Fettsäuren + 2-Ethyl-1-Hexanol → Fettsäure-2-ethylhexylester + Wasser.

Das Böhmit nimmt das entstehende Glycerol und Wasser auf. Anschließend kann das überschüssige 2-Ethyl-1-Hexanol durch Umesterung beispielsweise mit Biodiesel eliminiert werden.

## Patentansprüche

1. Lipasenformulierung, enthaltend mindestens eine Lipase in einem organischen Lösungsmittel und gemahlenes Aluminiumhydroxid (in Form von Böhmit (γ-AlO(OH)) mit einem mittleren Korndurchmesser kleiner 0,5 mm.

2. Lipasenformulierung nach Anspruch 1, **dadurch gekennzeichnet, dass** die Lipasenaktivität bei 5 bis 100 KLU/g liegt.

3. Lipasenformulierung nach einem der vorhergehenden Ansprüche, **dadurch gekennzeichnet, dass** auf 1 bis 5 ml Lipaselösung 1 bis 10 g gemahlenes Böhmit enthalten sind.

4. Lipasenformulierung nach einem der vorhergehenden Ansprüche, **dadurch gekennzeichnet, dass** als Lipase *Candida antarctica* (A-Lipase) enthalten ist.

5. Lipasenformulierung nach einem der vorhergehenden Ansprüche, **dadurch gekennzeichnet, dass** zusätzlich *Thermomyces lanuginosus* und/oder *Candida antarctica* (B-Lipase) und/oder *Aspergillus oryzae* enthalten ist.

6. Lipasenformulierung nach einem der vorhergehenden Ansprüche, **dadurch gekennzeichnet, dass** das gemahlene Böhmit einen mittleren Korndurchmesser kleiner 0,2 mm, bevorzugt kleiner 0,1 mm, besonders bevorzugt kleiner 0,07 mm aufweist.

7. Lipasenformulierung nach einem der vorhergehenden Ansprüche, **dadurch gekennzeichnet, dass** das Böhmit ein gemahlenes Böhmit ist.

8. Verwendung der Lipasenformulierung nach einem der Ansprüche 1 bis 7, für die Alkoholyse von Fetten, Diglyceriden und/oder Monoglyceriden.

9. Verwendung der Lipasenformulierung nach einem der Ansprüche 1 bis 7 zur Veresterung von Fettsäuren.

10. Verwendung der Lipasenformulierung nach einem der Ansprüche 1 bis 7 zur Umesterung von Fettsäuremethylester.

11. Verwendung der Lipasenformulierung nach einem der Ansprüche 1 bis 7 für die parallel ablaufenden Reaktionen Alkoholyse von Gemischen aus Tri-, Di- und Monoglyceriden und Veresterung von Fettsäuren.
